# EUROPÄISCHE PATENTSCHRIFT

(11) **EP 1 124 594 B1**
(45) Veröffentlichungstag und Bekanntmachung des Hinweises auf die Patenterteilung: **08.01.2003**
(21) Anmeldenummer: 99971334.0
(22) Anmeldetag: 02.11.1999
(51) Int. Cl.: A61L 31/14, A61L 31/16, A61L 31/02

(54) **IMPLANTAT MIT HOHLRÄUMEN, DIE THERAPEUTISCHE MITTEL ENTHALTEN**
IMPLANT WITH CAVITIES CONTAINING THERAPEUTIC AGENTS
IMPLANT POURVU DE CAVITES CONTENANT DES AGENTS THERAPEUTIQUES

(30) Priorität: 02.11.1998 DE 19850352; 01.12.1998 DE 19855421; 18.02.1999 DE 19907006; 09.03.1999 DE 19910188
(43) Veröffentlichungstag der Anmeldung: 22.08.2001
(73) Patentinhaber: AlCove Surfaces GmbH, 45966 Gladbeck (DE)
(72) Erfinder: BRANDAU, Wolfgang, D-48161 Münster (DE); FISCHER, Alfons, D-45239 Essen (DE); SAWITOWSKI, Thomas, D-45133 Essen (DE); SCHMID, Günter, D-42555 Velbert (DE)
(74) Vertreter: Gesthuysen, von Rohr & Eggert
(86) Internationale Anmeldenummer: EP9908346
(87) Internationale Veröffentlichungsnummer: WO00025841

(56) Entgegenhaltungen:
- WO-A-98/48851
- CA-A- 2 235 031
- DE-A- 19 537 872
- US-A- 5 769 883
- DUNN D S ET AL: "Anodized layers on titanium and titanium alloy orthopedic materials for antimicrobial activity applications" MATER MANUF PROCESS;MATERIALS AND MANUFACTURING PROCESSES 1992, Bd. 7, Nr. 1, 1992, Seiten 123-137, XP000884191
- PATENT ABSTRACTS OF JAPAN vol. 1999, no. 12, 29. Oktober 1999 (1999-10-29) & JP 11 181596 A (NAGOYA ALUMITE KK), 6. Juli 1999 (1999-07-06)
- SCHULTZE J W ET AL: "Regular nanostructured systems formed electrochemically: deposition of electroactive polybithiophene into porous silicon" ELECTROCHIMICA ACTA,GB,ELSEVIER SCIENCE PUBLISHERS, BARKING, Bd. 40, Nr. 10, 1. Juli 1995 (1995-07-01), Seiten 1369-1383, XP004019809 ISSN: 0013-4686

## Beschreibung

Die vorliegende Erfindung betrifft ein Implantat gemäß dem Oberbegriff des Anspruchs 1, Verfahren zur Herstellung eines Implantats gemäß dem Oberbegriff des Anspruchs 11 bzw. 12 sowie eine Verwendung eines Implantats gemäß dem Oberbegriff des Anspruchs 14.

Hier ist unter dem Begriff "Implantat" zunächst im engeren Sinne ein in den Körper eines Tieres oder eines Menschen zumindest vorübergehend einzusetzendes Element, das beispielsweise therapeutische, Stütz- und/oder Gelenkfunktionen ausüben kann, wie temporäre Implantate, beispielsweise sogenannte "Seeds", oder Stents zur Tumorbehandlung bzw. -therapie, Tracheal-Stents und dgl., zu verstehen. Im weiteren Sinne sind hierunter jedoch auch mit dem Körper von außen, insbesondere vorübergehend in Kontakt bringbare Elemente o. dgl. zu verstehen.

Implantate in Form von Stents werden beispielsweise zur Abstützung aufgeweiteter Gefäße eingesetzt. Diese röhrenförmigen Einsätze werden nach der Aufweitung verengter Gefäße eingeführt und anschließend radial aufgeweitet, so daß die Stents die Gefäßwandungen innenseitig abstützen.

Die Stents wachsen innerhalb von etwa ein bis drei Monaten in die Gefäßwände ein. Zur Vermeidung eines überschießenden Wachstums der Gefäßwände nach innen, was zu einer Restenose, also einer Wiederverengung, führen kann, können radioaktive Stents eingesetzt werden. Hierzu ist es bereits bekannt, eine Ionenimplantation vorzunehmen. Hierbei wird radioaktiver Phosphor (³²P) in vorhandene Stent-Oberflächen mittels eines Ionenstrahls implantiert. Weiter ist es bekannt, Nickel-Titan-Stents in einem Zyklotron o. dgl. mit Protonen zu beschießen, um das in gebräuchlichen Nickel/Titan-Legierungen enthaltene Titan zu radioaktivem Vanadium (⁴⁸V) zu aktivieren.

Sowohl die Ionenimplantation als auch die Protonenaktivierung zeichnen sich durch einen hohen technischen Aufwand aus, d. h. die Stents können praktisch nur in "Einzelanfertigung" hergestellt werden. Außerdem sind beide Methoden bislang auf wenige Herstellungsorte und wenige Radionuklide beschränkt.

Außerdem kann es sinnvoll sein, Medikamente möglichst lokal wirken zu lassen, um zum Beispiel eine Abstoßung des Implantats zu verhindern oder beispielsweise eine lokale Tumorbehandlung durchzuführen.

Aus der zur EP - A - 0 875 218 korrespondierenden CA - A - 2,235,031 ist bereits ein Stent bekannt, der in einer Ausführungsform einen nicht porösen Träger mit einer porösen Deckschicht aufweist. Die poröse Deckschicht ist aus gesinterten Metallpartikeln gebildet. Ein Medikament bzw. therapeutisches Mittel ist in den Poren der porösen Deckschicht aufgenommen und kann, wenn die poröse Deckschicht von einer beispielsweise auflösbaren oder durchlässigen Abdeckschicht überdeckt ist, im implantieren Zustand von dem Stent wieder abgegeben werden. Ggf. kann auch ein radioaktives Material als Medikament eingesetzt werden.

Bei dem bekannten Stent ist nachteilig, daß die gesinterten Metallpartikel der porösen Deckschicht sehr ungleichmäßige, undefinierte Poren bilden. Im Falle eines abzugebenden Medikaments wird dementsprechend nur ein verhältnismäßig undefiniertes Abgabeverhalten erreicht.

Wenn ein radioaktives Material in den Poren der Deckschicht aufgenommen wird, besteht die Gefahr, daß aufgrund der unregelmäßigen Poren mit undefinierten Öffnungen das radioaktive Material unkontrolliert und ungewollt entweicht. Die optional vorgesehene Beschichtung der Deckschicht bietet diesbezüglich keinen ausreichenden Schutz.

Die Festigkeit und mechanische Belastbarkeit der aus zusammengesinterten Metallpartikeln gebildeten Deckschicht sind insbesondere bei einer Verformung des Stents nicht sehr gut. Insbesondere besteht die Gefahr, daß sich zumindest einzelne Metallpartikel aus der Deckschicht lösen. Zudem besteht die Gefahr einer Segmentierung der Deckschicht, insbesondere beim radialen Aufweiten des Stents. Hierbei besteht jeweils die Gefahr, daß beispielsweise der Blutkreislauf Teile der Deckschicht an andere Körperstellen mit unerwünschten Folgen transportiert. Dieses Risiko ist besonders hoch bei Einsatz von radioaktivem Material, das als Medikament bzw. therapeutisches Mittel in der porösen Deckschicht fixiert bleiben sollte.

Außerdem steht bei metallischen Implantaten insbesondere Nickel im Verdacht, ein übermäßiges Zellwachstum, insbesondere im Bereich um ein eingesetztes Implantat herum, zumindest zu fördern. Weitere Metalle, die ebenfalls von Körperflüssigkeiten, wie Blut, aus Metalloberflächen - wenn auch nur in geringem Maße - gelöst werden können, werden außerdem zunehmend für unerwünschte Folgen oder zumindest nicht überschaubare Reaktionen im Körper verantwortlich gemacht.

Die Veröffentlichung "Anodized layers on titanium and titanium alloy orthopedic materials for antimicrobial activity applications" von D. S. Dunn et al., veröffentlicht in "Materials & Manufacturing Processes", Band 7, Nr. 1, 1992, Seiten 123 bis 137, die den Ausgangspunkt für Anspruch 1 bildet, offenbart orthopädische Implantate aus Titan bzw. einer Titanlegierung mit einer anodisch oxidierten Oberflächen-Deckschicht aus Titandioxid, die Poren in einer Größe von 0,1 bis 10 µm zur Aufnahme eines Antibiotikums aufweist. Die Poren sind sehr ungleichmäßig ausgebildet, so daß sich ein undefiniertes Verhalten hinsichtlich der Aufnahme und des Rückhaltens oder der gezielten Abgabe eines therapeutischen Mittels ergibt. Zudem können Blutbestandteile verhältnismäßig leicht in die relativ großen Poren eindringen und ungewollt die Poren zusetzen und/oder ein therapeutisches Mittel aus den Poren herauslösen.

Die US 5,769,883 A1 beschreibt spulenförmige Stents aus biodegardierbarem Material mit Poren in der biodegradierbaren Matrix, die mit Medikamenten gesättigt ist.

Die nachveröffentlichte WO 98/48851 A2 betrifft auf der Oberfläche radioaktiv beschichtete Stents. Zur Herstellung wird ein nicht radioaktiver Stent in eine Lösung, die ein radioaktives Isotop in ionischer Form enthält, getaucht und das Isotop dann chemisch, insbesondere reduktiv, durch chemische Fällung oder elektrochemisch, abgeschieden. Beispielsweise wird eine metallische Stentoberfläche in einer radioaktiven Phosphorsäurelösung anodisch oxidiert. Die Ausbildung definierter Hohlräume zur Aufnahme eines therapeutischen Mittels ist nicht offenbart.

Die DE 31 01 679 C2, die den Ausgangspunkt des Anspruchs 14 bildet, beschreibt Implantate mit einer relativ porösen Deckschicht aus anodisch oxidiertem Aluminium. Die Aluminiumoxidschicht dient dazu, eine gegenüber Körpergewebe verträgliche Oberfläche zu erzeugen.

Der vorliegenden Erfindung liegt die Aufgabe zugrunde, ein Implantat, Verfahren zur Herstellung eines Implantats und eine Verwendung eines Implantats einzugeben, so daß ein insbesondere als Stent ausgebildetes Implantat verhältnismäßig einfach herstellbar ist, wobei ein therapeutisches Mittel vom Implantat aufnehmbar und - falls gewünscht - im implantierten Zustand lokal wieder abgebbar ist, wobei insbesondere bedarfsweise eine sichere Fixierung von Radionukliden auf bzw. in der Oberfläche ermöglicht wird.

Die obige Aufgabe wird durch ein Implantat gemäß Anspruch 1, durch ein Verfahren gemäß Anspruch 11 oder 12 oder durch eine Verwendung gemäß Anspruch 14 gelöst. Vorteilhafte Weiterbildungen sind Gegenstand der Unteransprüche.

Insbesondere weist die Deckschicht eine Vielzahl von definierten Hohlräumen mit separaten Öffnungen zur Oberfläche der Deckschicht zur Aufnahme mindestens eines therapeutischen Mittels auf. Unter dem Begriff "Hohlräume" sind hier auch definierte Fehlstellen in Kristallgefügen oder dergleichen zu verstehen, die zur Aufnahme eines therapeutischen Mittels geeignet sind.

Durch die Ausbildung definierter und insbesondere voneinander getrennter Hohlräume in der Deckschicht sind im Gegensatz zum Stand der Technik sehr genaue Mengen eines therapeutischen Mittels in die Hohlräume einlagerbar, in den Hohlräumen bei Bedarf fixierbar und - falls gewünscht - im implantierten Zustand unter definierten Bedingungen, wie mit einer gewünschten Abgaberate, wieder abgebbar.

Unter dem Begriff "therapeutisches Mittel" sind in der vorliegenden Patentanmeldung Medikamente im weitesten Sinne, gegebenenfalls auch radioaktives Material oder sonstige therapeutische Stoffe zu verstehen. Insbesondere kommen auch alle in der EP - A - 0 875 218 genannten, als "medication" bezeichneten therapeutischen Mittel bzw. Rezeptoragonisten, Rezeptorantagonisten, Enzyminhibitoren, Neurotransmitter, Zytostatika, Antibiotika, Hormone, Vitamine, Stoffwechselsubstrate, Antimetabolite, Diuretika und dergleichen als therapeutisches Mittel in Betracht.

Außerdem ist ein vorschlagsgemäßes Implantat mit einem Träger und einer Deckschicht versehen, wobei die Deckschicht vorzugsweise zumindest im wesentlichen aus Aluminium-, Magnesium-, Tantal-, Eisen- und/oder Wolframoxid besteht. Eine derartige Deckschicht ist verhältnismäßig einfach herstellbar, beispielweise durch elektrolytische Abscheidung und Oxidierung, und bildet eine hochgradig chemisch und mechanisch stabile, insbesondere sehr dichte Beschichtung des Trägers. Diese Beschichtung kann zumindest weitgehend ein (ionisches) Lösen von Nickel oder anderen Metallen aus dem Träger verhindern. So kann ein durch die gelösten Metalle induziertes übermäßiges Zellwachstum in der Umgebung bzw. in dem Anlagebereich des Implantats zumindest minimiert werden.

Eine einfache Ausbildung der Hohlräume in der Deckschicht wird vorzugsweise durch anodische Oxidierung einer Oberflächenschicht, die Teile des Trägers oder einer darauf abgeschiedenen Beschichtung sein kann, erreicht.

So lassen sich auf einfache Weise gleichförmige Hohlräume definierter Dimensionen ausbilden. Eine sehr einfache Herstellung von hochgradig gleichförmigen Hohlräumen wird vorzugsweise dadurch ermöglicht, daß als Deckschicht eine Aluminiumoxidschicht elektrolytisch auf der Fläche Oberfläche des Trägers gebildet wird. Bei einer derartigen künstlichen Oxidierung von Aluminium (Anodisierung) können in Abhängigkeit von der angelegten Spannung definierte Hohlräume gebildet werden. Neben Aluminiumoxid eignen sich hierfür insbesondere alle sogenannten Ventilmetalloxide, beispielsweise Wolframoxide. Darüber hinaus kommt auch Magnesiumoxid in Betracht.

Durch Variation der elektrischen Spannung bei der Anodisierung können der Durchmesser der Hohlräume und die Flächendichte der Hohlräume, d.h. die Anzahl der Hohlräume pro Fläche, variiert werden. Die Länge der Hohlräume hängt von der Dauer der Anodisierung ab. Folglich kann die Form der Hohlräume in weiten Bereichen gesteuert werden, so daß eine beispielsweise im Hinblick auf ein gewünschtes Abgabeverhalten (Abgabegeschwindigkeit, Abgabemenge) optimierte Form der Hohlräume auf einfache Weise realisierbar ist. Beispielsweise sind die Hohlräume zumindest im wesentlichen röhrenartig ausgebildet und erstrecken sich von der Oberfläche der Deckschicht in wesentlichen senkrecht in das Innere der Deckschicht, wobei der Querschnitt der Hohlräume und/oder deren Öffnung im Durchmesser bzw. in der Fläche abschnittsweise reduziert sind, um gewünschte Eigenschaften zu erhalten.

Je nach Anwendungsfall und Bedarf können auch mehrere therapeutische Mittel von den Hohlräumen aufgenommen sein, die beispielsweise nacheinander und/oder mit unterschiedlicher Abgabegeschwindigkeit im implantierten Zustand wieder abgegeben werden. So können beispielsweise therapeutische Mittel unterschiedlicher Molekülgröße in unterschiedlich, angepaßt dimensionierten Hohlräumen der Deckschicht des Implantats aufgenommen sein. Bei Bedarf ist es auch möglich, die Hohlräume bzw. deren Öffnungen zur Oberfläche der Deckschicht hin im Verhältnis zu den üblicherweise in Körperflüssigkeiten, wie im Blut, befindlichen Bestandteilen, insbesondere Proteinen, klein auszubilden mit der Folge, daß ein ansonsten auftretendes Lösen bzw. Auswaschen des in den Hohlräumen befindlichen therapeutischen Mittels durch makromolekulare Blutbestandteile oder dergleichen nicht auftritt, da diese nicht in die Hohlräume eindringen können.

Die Integration der Hohlräume in die Deckschicht des Trägers ermöglicht einen verhältnismäßig dünnen Aufbau mit dementsprechend geringer Neigung zur Segmentierung, also einen Aufbau mit günstigen mechanischen Eigenschaften.

Die Ausbildung der Hohlräume an bestimmten Stellen mit verhältnismäßig geringer Flächenausdehnung relativ zur Flächenausdehnung der Deckschicht führt zu dem Vorteil, daß die mechanischen Eigenschaften der Deckschicht im wesentlichen nur von dem Material der Deckschicht und nicht von dem therapeutischen Mittel oder dergleichen in den Hohlräumen abhängen. Dementsprechend werden einerseits der Einsatz von einer hinsichtlich der gerade bei Stents großen mechanischen Beanspruchung optimierten Deckschicht und andererseits die Verwendung von hinsichtlich der Behandlung optimal geeigneten, therapeutischen Mitteln ermöglicht.

Grundsätzlich können die Hohlräume untereinander verbunden sein. Vorzugsweise sind die Hohlräume jedoch gerade im Hinblick auf eine niedrige Bauhöhe bzw. Dicke der Deckschicht getrennt voneinander ausbildet.

Insbesondere bei getrennter Ausbildung der Hohlräume ist es möglich, ein therapeutisches Mittel oder mehrere therapeutische Mittel in den Hohlräumen in unterschiedlicher Konzentration bzw. Menge oder mit unterschiedlichem Abgabeverhalten anzuordnen, um beispielsweise eine gewünschte zeitlich und/oder räumlich inhomogene Dosisverteilung mit beispielsweise an den Enden eines Stents erhöhter Dosis zu erreichen.

Das Einbringen des therapeutischen Mittels und/oder von Komplexbildnern bzw. Bindungspartnern in die Hohlräume erfolgt vorzugsweise dadurch, daß die Hohlräume der Deckschicht evakuiert und anschließend das therapeutische Mittel oder die Komplexbildner bzw. Bindungspartner zugegeben wird bzw. werden, das bzw. die dann von den Hohlräumen aufgenommen bzw. quasi in diese eingesaugt wird bzw. werden. Bedarfsweise wird dies beispielsweise für Hohlräume in bestimmten Oberflächenbereichen, insbesondere Endbereichen des Implantats wiederholt, um eine lokale Erhöhung der Menge an aufgenommenem therapeutischen Mittel zu erreichen.

Alternativ oder zusätzlich kann das Einbringen des therapeutischen Mittels oder von Bindungspartnern in die Hohlräume mittels Ultraschall, der bei Eintauchen des Implantats in das einzubringende Mittel in den Hohlräumen vorhandene Luft oder sonstige Gase entweichen läßt, erreicht bzw. unterstützt werden.

Ein weiterer Aspekt der vorliegenden Erfindung liegt darin, das therapeutische Mittel bzw. die therapeutischen Mittel in den Hohlräumen bedarfsgerecht, beispielsweise ionisch über Wasserstoffbrücken, über Komplexbildner, durch Van der Waals-Kräfte, oder dergleichen, zu fixieren bzw. zu binden, um eine gewünschte Abgabe bzw. Freisetzung des therapeutischen Mittels bzw. der therapeutischen Mittel zu erreichen. Ebenso sind Bindungen möglich, die chemisch bzw. enzymatisch in biologischen Systemen gespalten bzw. aufgebrochen werden und dadurch die Freisetzung bewirken. Gewünschte Eigenschaften der Hohlräume können verhältnismäßig einfach durch chemische Modifizierung der Wandungen der Hohlräume erreicht werden, insbesondere dadurch, daß für das jeweilige therapeutische Mittel geeignete Bindungspartner auf den Wandungsoberflächen chemisch fixiert werden.

Schließlich ist darauf hinzuweisen, daß das vorschlagsgemäße Implantat auch mit nach außen offenen Hohlräumen in der Deckschicht versehen sein kann, wobei die Größe der Hohlräume so gewählt sein kann, daß Zellen oder Teile von Zellen von sich an das Implantat anschließendem Körpergewebe in die Hohlräume einwachsen können und so beispielsweise eine sehr sichere Verankerung des Implantats im Körper erreicht werden kann.

Außerdem besteht die Möglichkeit, die Deckschicht bzw. die Öffnungen der Hohlräume mit einer Abdeckschicht als Schutzschicht zu überdecken. Diese Abdeckschicht kann sehr dünn ausgeführt sein, da sie im wesentlichen nur dazu dient, gewünschte Oberflächeneigenschaften oder eine Abdeckung des Materials der Deckschicht zu erreichen. Die Abdeckschicht kann je nach Anwendungsfall - beispielsweise derart ausgebildet sein, daß sie sich im Körper, beispielsweise aufgrund der Körpertemperatur, einer künstlichen Erwärmung, chemischer oder enzymatischer Einwirkungen von Flüssigkeiten oder körpereigenen Stoffen, auflöst bzw. von der Oberfläche der Deckschicht löst oder daß sie für ein in den Hohlräumen aufgenommenes therapeutisches Mittel durchlässig ist. Insbesondere kann die Abdeckschicht wie die in der EP - A - 0 875 218 offenbarte Beschichtung des porösen Materials ausgebildet sein.

Bei der insbesondere vorgesehenen Verwendung von radioaktivem Material als therapeutisches Mittel liegt ein wesentlicher Aspekt der vorliegenden Erfindung darin, daß das radioaktive Material nicht großflächig, sondern nur an einzelnen Stellen lokalisiert bzw. in der Deckschicht eines Trägers angeordnet wird. Die Deckschicht kann grundsätzlich durch eine Oberflächenschicht, also einen oberen Teil, des Trägers oder insbesondere durch eine auf die Oberfläche des Trägers aufgebrachte Schicht bzw. Beschichtung gebildet sein. So ist es möglich, die Hohlräume bzw. deren Öffnungen zur Oberfläche der Deckschicht hin im Verhältnis zu den üblicherweise im Blut befindlichen Bestandteilen, insbesondere Proteinen, klein auszubilden mit der Folge, daß das bei einem großflächigen Aussetzen von radioaktivem Material ansonsten auftretende Lösen bzw. Abtragen des radioaktiven Materials durch makromolekulare Blutbestandteile nicht auftritt, da diese nicht in die Hohlräume eindringen können.

Ein weiterer Vorteil der Hohlräume liegt in der Schaffung einer sehr großen inneren Oberfläche durch die Hohlraumwandlungen. Diese innere Oberfläche stellt eine wesentlich größere Oberfläche als die äußere Oberfläche der Deckschicht dar und gestattet dementsprechend eine insbesondere festere bzw. stärkere Bindung von mehr radioaktivem Material als herkömmliche Mehrschichtaufbauten.

Ein weiterer Vorteil der Anordnung des radioaktiven Materials in den Hohlräumen liegt in der bedarfsweise unterschiedlichen Konzentration des radioaktiven Materials, um eine gewünschte inhomogene räumliche Dosisverteilung mit beispielsweise an den Enden eines Stents erhöhter Dosis zu erreichen, indem die Hohlräume in manchen Oberflächenbereichen mit unterschiedlichen Mengen an radioaktivem Material "gefüllt" werden.

Vorzugsweise sind die Hohlräume zumindest im wesentlichen röhrenartig ausgebildet und erstrecken sich von der Oberfläche der Deckschicht im wesentlichen senkrecht in das Innere der Deckschicht, wobei der Querschnitt der Hohlräume und/oder deren Öffnung vorzugsweise so klein dimensioniert ist, daß zumindest die meisten der üblicherweise im Blut vorhandenen Proteine aufgrund ihrer Molekülgröße nicht in die Hohlräume, insbesondere wenn diese nur teilweise gefüllt sind, eindringen können. Dementsprechend kann das in den Hohlräumen befindliche radioaktive Material nicht vom Blut fortgetragen werden.

Die Verwendung einer Oxidschicht, insbesondere aus Aluminiumoxid, als Deckschicht führt zu dem zusätzlichen Vorteil, daß die Oxidschicht in Flüssigkeit einer Art Quellung unterliegt, was zu einem Verschließen bzw. weiterem Verringern der Öffnungsfläche der Öffnungen der Hohlräume in der Deckschicht führt, wodurch das Eindringen der relativ großen Proteine im Blut weiter erschwert bzw. verhindert wird. Diese Quellung ist natürlich zu berücksichtigen, wenn sich die Öffnungen beispielsweise bei gewollter Abgabe eines sonstigen therapeutischen Mittels eben gerade nicht verschließen sollen.

Das Einbringen des radioaktiven Materials und/oder von Komplexbildnern in die Hohlräume kann vorzugsweise dadurch erfolgen, daß die Hohlräume evakuiert und anschließend das radioaktive Material oder die Komplexbildner zugegeben werden, die dann von den Hohlräumen aufgenommen bzw. quasi in diese eingesaugt werden. Bedarfsweise wird dies beispielsweise für Hohlräume in bestimmten Oberflächenbereichen, insbesondere Endbereichen des Implantats, wiederholt, um eine lokale Erhöhung der Radioaktivität zu erreichen.

Ein unabhängiger, weiterer Aspekt der vorliegenden Erfindung liegt darin, das radioaktive Material, also eine insbesondere vorbestimmte Menge eines Radionuklids oder verschiedener Radionuklide, vorzugsweise in den Hohlräumen über Komplexbildner, wie Amine, Phosphine, Carboxylate und/oder Thiole, zu fixieren. Insbesondere sind Thiole als Komplexbildner und beispielsweise Technetium und Rhenium als radioaktives Material vorgesehen, da Technetium(V)- und Rhenium(V)-Verbindungen mit schwefelhaltigen Liganden Metallkomplexe bilden, die eine extrem hohe in vivo Stabilität aufweisen. Radioaktives Kupfer als anderes Beispiel wird hingegen besser über Carboxylate gebunden. Mit Hilfe von Komplexbildnern lassen sich also insbesondere radioaktive Kationen (Metalle) chemisch, insbesondere in den Hohlräumen oder Poren der Deckschicht, sehr fest binden. Die Komplexbildner selbst sind dabei vorzugsweise an den Wandungen der Hohlräume, insbesondere durch Silanisierung, fixiert bzw. gebildet, so daß der Komplex insgesamt auf der Oberfläche bzw. in der Deckschicht des Trägers fixiert ist.

Alternativ kann auch eine Bindung von radioaktiven Anionen (Nichtmetalle), beispielsweise Iod, durch Komplexbildung mit entsprechenden Komplexbildnern bzw. mit entsprechenden Bindungspartnern, beispielsweise in den Hohlräumen fixierten Metallen, wie Edelmetalle, insbesondere Silber, vorgesehen sein.

Ein unabhängiger, weiterer wesentlicher Aspekt der vorliegenden Erfindung liegt darin, daß verschiedene Radionuklide mit dementsprechend unterschiedlichen Halbwertszeiten und Emissionsenergien, wie ¹⁸⁶Re (T_{1/2} = 90 h, E_{βmax} = 1,071 MeV) und ¹⁸⁸Re (T_{1/2} = 16,7 h, E_{βmax} = 2.116 MeV), als Mischung bzw. Gemenge zusammen in vorbestimmten Mengen und Verhältnissen verwendet werden. So kann eine für die jeweilige Anwendung optimale Dosisverteilung sowohl in räumlicher als auch in zeitlicher Hinsicht erreicht werden. Die Fixierung unterschiedlicher Radionuklide wird gerade durch die Bereitstellung der Hohlräume zur Aufnahme der Radionuklide ermöglicht, da die mechanischen Eigenschaften der Radionuklide bzw. der mit den Radionukliden gebildeten Verbindungen in den Hohlräumen aufgrund der verhältnismäßig geringen Ausdehnung der Hohlräume fiir die mechanischen Eigenschaften der Deckschicht eine allenfalls geringe Rolle spielen, so daß auch ansonsten für großflächige Beschichtungen nicht verwendbare Radionuklide oder Radionuklid-Verbindungen in die Hohlräume aufnehmbar und darin fixierbar sind.

Außerdem besteht die Möglichkeit, die Deckschicht bzw. die Öffnungen der Hohlräume mit einer Abdeckschicht, beispielsweise aus Gold, als Schutzschicht zu überdecken. Diese Abdeckschicht kann sehr dünn ausgeführt werden, da sie im wesentlichen nur dazu dient, gewünschte Oberflächeneigenschaften oder eine Abdeckung des Materials der Deckschicht zu erreichen, wobei im Gegensatz zum Stand der Technik eine Verhinderung des Kontakts von Blut mit dem radioaktiven Material nachrangig ist, da das radioaktive Material in den Hohlräumen chemisch fixiert und daher durch die Hohlräume ohnehin bereits geschützt ist. Des weiteren kann eine wesentlich bessere Haftung der Abdeckschicht auf der Deckschicht aufgrund der Freiheit in der Materialwahl erreicht werden, da die mechanischen und chemischen Eigenschaften der Deckschicht eben gerade nicht durch das verwendete radioaktive Material wesentlich beeinflußt werden.

Nachfolgend wird die vorliegende Erfindung anhand der Zeichnung bevorzugter Ausführungsbeispiele näher erläutert. Es zeigt:
- Fig. 1: eine schematische Darstellung eines vorschlagsgemäßen, als Stent ausgebildeten Implantats im nicht aufgeweiteten Zustand;
- Fig. 2: eine schematische Darstellung des Stents gemäß Fig. 1 im radial aufgeweiteten Zustand;
- Fig. 3: einen schematischen Querschnitt des in ein Gefäß eingesetzten und radial aufgeweiteten Stents gemäß Fig. 2;
- Fig. 4: eine ausschnittsweise Vergrößerung eines Trägers mit einer zugeordneten Deckschicht mit mehreren Hohlräumen des Implantats;
- Fig. 5 a, b, c: ausschnittsweise Vergrößerungen von Hohlräumen der Deckschicht gemäß Fig. 4 und einer zugeordneten Abdeckschicht; und
- Fig. 6, 7: elektronenmikroskopische Aufnahmen einer Aluminiumoxidschicht mit Hohlräumen in unterschiedlichen Vergrößerungen.

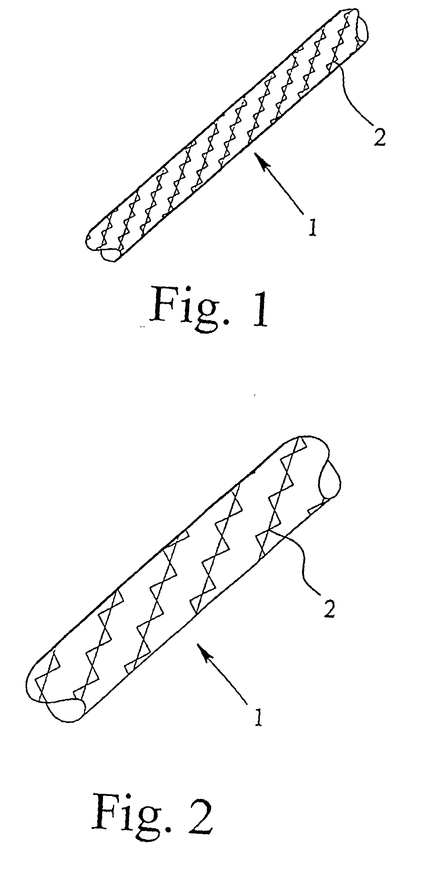

Ein vorschlagsgemäßes Implantat 1 ist in den Fig. 1 bis 3 schematisch dargestellt. Das Implantat 1 weist im vorliegenden Ausführungsbeispiel die Form eines Stents auf, also eines im wesentlichen röhrenförmigen Einsatzes für Gefäße, wie den Fig. 1 und 2 zu entnehmen ist.

Das Implantat 1 bzw. der Stent weist einen vorzugsweise metallischen oder metallisierten Träger 2 auf. Der Träger 2 ist hier verformbar ausgebildet, so daß der Stent radial aufgeweitet werden kann. Die Fig. 1 zeigt den Stent im nicht aufgeweiteten Zustand, Fig. 2 im radial aufgeweiteten Zustand.

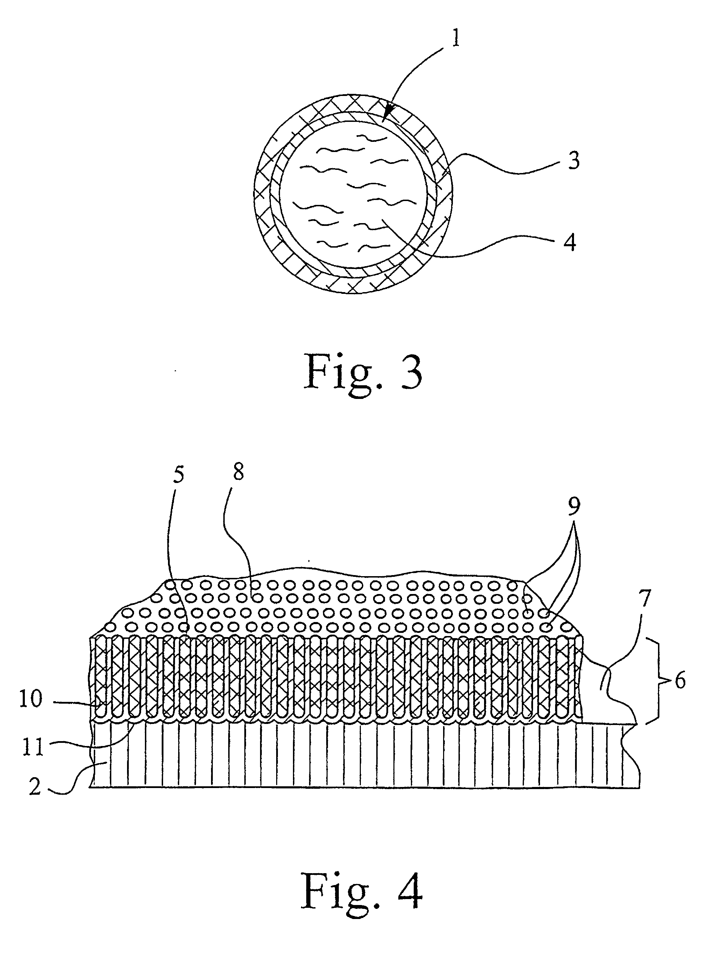

Die Fig. 3 zeigt den Stent im radial aufgeweiteten Zustand in einem Gefäß 3, wobei der Stent bzw. das Implantat 1 mit seiner Außenseite an der Innenseite der Gefäßwandung anliegt und so das beispielsweise dilatierte Gefäß 3 innen abstützt. Das Gefäß 3 stellt also Körpergewebe dar, das mit dem Träger 2 in Kontakt steht. Weiter steht der Träger 2 bzw. das Implantat 1 mit Körperflüssigkeiten, wie Blut 4, die beispielsweise durch das Gefäß 3 und den Stent hindurchströmen, in Kontakt.

Dem Träger 2 ist mindestens ein therapeutisches Mittel bzw. Medikament 5 zugeordnet, das auf bzw. im Träger 2 fixiert ist, wie der schematischen ausschnittsweisen Vergrößerung eines Oberflächenbereichs des Trägers 2 mit einer zugeordneten, teilweise weggeschnittenen Deckschicht 6 gemäß Fig. 4 zu entnehmen ist. Hinsichtlich des therapeutischen Mittels 5 wird insbesondere auf die obige Definition verwiesen.

Die Deckschicht 6 ist hier vorzugsweise auf der gesamten Oberfläche 7 des Trägers 2 aufgebracht, beispielsweise durch elektrolytische Abscheidung und Oxidierung oder Plasmabeschichtung. Alternativ könnte die Deckschicht 6 jedoch auch durch eine Oberflächenschicht des Trägers 2, abhängig vom Material des Trägers 2 und der gewünschten Zusammensetzung und Ausbildung der Deckschicht 6, gebildet sein.

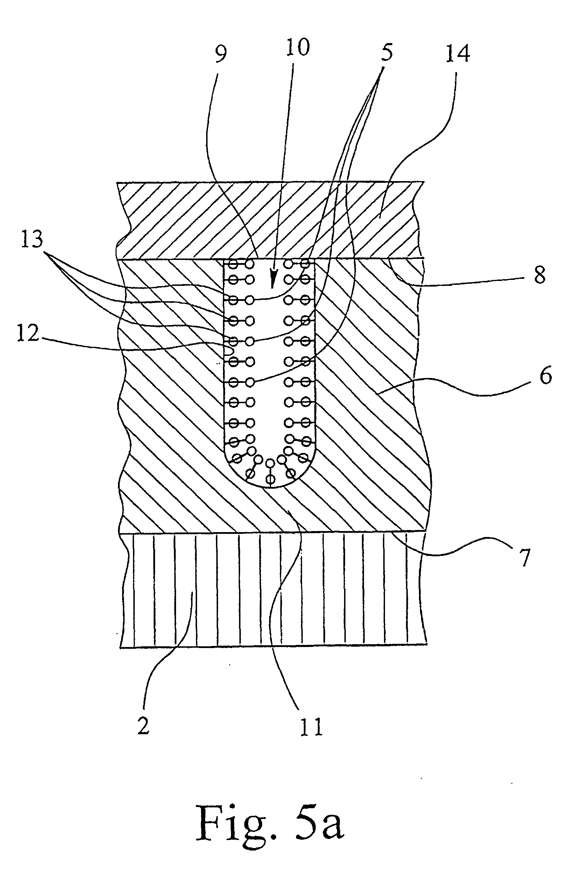

Die Deckschicht 6 weist auf ihrer dem Träger 2 abgewandten Oberfläche 8 eine Vielzahl von verteilten, zueinander beabstandeten Öffnungen 9 und sich daran anschließenden Hohlräumen 10 auf. Das therapeutische Mittel 5, auf das später noch näher eingegangen wird, ist in den Hohlräumen 10 aufgenommen und ggf. chemisch fixiert, wie später noch anhand Fig. 5a näher erläutert.

Die Hohlräume 10 sind hier im wesentlichen röhrenförmig ausgebildet und jeweils endseitig geschlossen. Sie erstrecken sich ausgehend von der Oberfläche 8 der Deckschicht 6 im wesentlichen senkrecht zum Träger 2 hin.

Insbesondere erstrecken sich die Hohlräume 10 weder bis zur Oberfläche 7 des Trägers 2 noch in den Träger 2 hinein, sondern enden jeweils blind in der Deckschicht 6 und sind durch eine Sperrschicht 11 der Deckschicht 6 von der Oberfläche 7 des Trägers 2 getrennt. Hierdurch wird eine zumindest weitgehende Abdichtung der gesamten Oberfläche 7 des Trägers 2 gegenüber Körpergeweben und -flüssigkeiten erreicht. Wichtig ist dabei eine hohe chemische Stabilität der Deckschicht 6 im Körper.

Die Hohlräume 10 sind hier im wesentlichen kreiszylindrisch ausgebildet. Sie könnten jedoch auch einen polygonalen Querschnitt oder eine unregelmäßige Querschnittsform aufweisen. Die Hohlräume 10 erstrecken sich hier im wesentlichen parallel zueinander und sind voneinander getrennt, ohne daß die Hohlräume 10 untereinander verbunden sind. Dies ist jedoch nicht unbedingt erforderlich; ggf. könnten in der Deckschicht 6 auch Verbindungen zwischen den Hohlräumen 10 untereinander bestehen.

Die Deckschicht 6 besteht vorzugsweise aus Aluminiumoxid, das insbesondere elektrolytisch auf der Oberfläche 7 des Trägers 2 abgeschieden bzw. gebildet wird. Bei der elektrolytischen Oxidierung kann der Durchmesser der Öffnungen 9 bzw. der Hohlräume 10 sehr einfach durch entsprechende Einstellung der angelegten Spannung verändert werden. Hierbei ergibt sich etwa ein Durchmesser von 1,2 bis 1,4 nm pro 1 V anodischer Spannung.

Die Deckschicht 6 bzw. nicht oxidiertes Deckschichtmaterial, wie Aluminium, kann alternativ beispielsweise durch Plasmabeschichten auf die Oberfläche 7 des Trägers 2 aufgebracht und ggf. anschließend oxidiert werden. Dies ist insbesondere dann vorteilhaft, wenn nur eine außenseitige Beschichtung gewünscht ist; eine zusätzliche innenseitige Beschichtung ist auf diese Weise jedoch auch möglich.

Die Herstellung der Deckschicht 6 ist jedoch nicht auf die voranstehenden Beispiele beschränkt, beispielsweise könnte auch eine Oxidierung einer entsprechenden Oberflächenschicht des Trägers 2 in Betracht kommen. Des weiteren ist das Material für die Deckschicht 6 nicht auf Aluminiumoxid beschränkt, sondern beispielsweise ist auch Magnesiumoxid einsetzbar. Wesentlich sind die mechanischen Eigenschaften der resultierenden Deckschicht 6 und die Ausbildung der Hohlräume 10 zur Aufnahme des therapeutischen Mittels 5.

Die schematische, vergrößerte Schnittdarstellung eines Hohlraums 10 gemäß Fig. 5a veranschaulicht die mögliche Fixierung des therapeutischen Mittels 5 in den Hohlräumen 10 der Deckschicht 6. Die Wandung 12 des Hohlraums 10 ist beispielsweise mit Reaktionspartnern, wie Komplexbildnern 13, die beispielsweise durch Silanisierung in den Hohlräumen 10 bzw. an deren Wandungen 12 gebunden sind, versehen.

Anstelle der in Fig. 5a beispielhaften Komplexbildner 13 können die Wandungen 12 der Hohlräume 10 bei Bedarf auch mit anderen, eine gewünschte Bindung des therapeutischen Mittels 5 hevorrufenden Bindungspartnern versehen sein. Alternativ ist vorzugsweise mindestens ein therapeutisches Mittel 5 von den Hohlräumen 10 aufgenommen, ohne daß es darin gebunden bzw. fixiert ist. Insbesondere in diesem Fall ggf. aber auch ansonsten ist vorzugsweise auf der Oberfläche 8 der Deckschicht 6 eine Abdeckschicht 14 vorgesehen, die auch die Hohlräume 10 bzw. deren Öffnungen 9 überdeckt.

Die Abdeckschicht 14 dient insbesondere dazu, ein vorzeitiges Entweichen bzw. Freisetzen des therapeutischen Mittels 5 aus den Hohlräumen 10, also vor dem Implantieren des Implantats 1, zu verhindern. Jedoch kann die Abdeckschicht 14, insbesondere wenn es sich bei dem therapeutischen Mittel 5 um radioaktives Material handelt, auch einer Verhinderung eines unmittelbaren Kontakts von Körpergewebe und/oder -flüssigkeiten mit dem therapeutischen Mittel 5 dienen. Da die Gesamtfläche der Öffnungen 9 vorzugsweise kleiner, insbesondere wesentlich kleiner, als die Kontaktfläche der Oberfläche 8 der Deckschicht 6 mit der Abdeckschicht 14 ist, kann die Abdeckschicht 14 unabhängig von dem therapeutischen Mittel 5, je nach gewähltem Material für die Deckschicht 6 und die Abdeckschicht 14, sehr gut auf der Deckschicht 6 haften. Vorzugsweise bilden die Wandungen 12 der Hohlräume 10 eine im Verhältnis zur Oberfläche 8 der Deckschicht 6 wesentlich größere innere Oberfläche insbesondere bei gewünschter Fixierung des therapeutischen Mittels in den Hohlräumen 10.

Wesentlich ist, daß die Deckschicht 6 und die gegebenenfalls vorgesehene Abdeckschicht 14 so dimensioniert und ausgebildet sind, daß eine Segmentierung, beispielsweise bei der radialen Aufweitung des Stents, mit Sicherheit ausgeschlossen ist. Hierzu beträgt die Dicke der Deckschicht 6 vorzugsweise weniger als 1,5 µm, vorzugsweise maximal 200 nm und insbesondere 30 nm bis 150 nm. Jedoch kann die Dicke der Deckschicht insbesondere zur Aufnahme größerer Volumen in den Hohlräumen 10 beispielsweise auch bis zu 150 µm betragen.

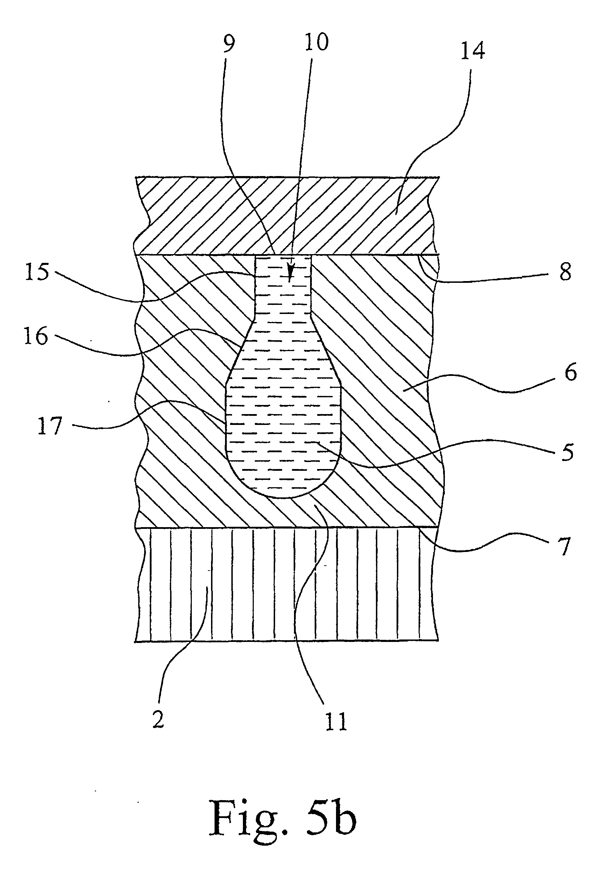

Fig. 5b zeigt in einer zu Fig. 5a korrespondierenden, ausschnittweisen Schnittdarstellung eine alternative Ausführungsform mit modifizierten Hohlräumen 10. Hier sind die Hohlräume 10 in einem Schnitt senkrecht zur Haupterstreckungsebene der Deckschicht 6 etwa flaschenförmig ausgebildet bzw. weisen jeweils einen verengten Abschnitt 15 im Bereich der Öffnung 9, einen sich auf der der Öffnung 9 entgegengesetzten Seite an den Abschnitt 15 anschließenden Übergangsabschnitt 16 mit zunehmenden Querschnitt und einen sich schließlich daran anschließenden Endabschnitt 17 mit dem größten Querschnitt bzw. Durchmesser auf. Bei diesem Ausführungsbeispiel begrenzt der im Querschnitt bzw. Durchmesser verringerte Abschnitt 15 die Abgaberate bzw. -geschwindigkeit, mit der das therapeutische Mittel 5 aus den Hohlräumen 10 im implantierten Zustand bei entfernter bzw. durchlässiger Abdeckschicht 14 abgegeben wird. Je nach Dimensionierung - bei elektrolytischer Anodisierung durch Variation der Spannung - der Hohlräume 10 kann somit eine gewünschte Abgaberate erreicht werden.

Bei Bedarf kann die Reihenfolge der in Fig. 5b beispielhaft dargestellten Abschnitte 15 bis 17 der Hohlräume 10 auch umgekehrt werden, so daß der den größten Durchmesser bzw. Querschnitt aufweisende Abschnitt 17 sich zur Oberfläche 8 hin öffnet, um eine zunächst sehr starke bzw. hohe Abgaberate und anschließend eine verringerte Abgaberate zu erreichen. In jedem Fall kann durch die Form bzw. Dimensionierung der Hohlräume 10 eine gewünschte zeitliche und ggf. auch räumliche Verteilung der Dosis an abgegebenem bzw. freigesetztem therapeutischen Mittel 5 festgelegt werden. Wesentlich ist dabei die definierte Ausbildung der Hohlräume 10.

In Fig. 5b ist beispielhaft angedeutet, daß ein einziges therapeutisches Mittel 5 von den Hohlräumen 10 aufgenommen ist. Bedarfsweise können auch verschiedene therapeutische Mittel 5, beispielsweise geschichtet, von den Hohlräumen 10 aufgenommen sein, um ein sukzessives Freisetzen der verschiedenen therapeutischen Mittel 5 zu erreichen. Alternativ oder zusätzlich können auch verschiedene therapeutischen Mittel 5 in beispielsweise unterschiedlich ausgebildeten und/oder mit unterschiedlichen Bindungspartnern versehenen Hohlräumen 10 der Deckschicht 6 aufgenommen sein, um eine gegebenenfalls gleichzeitige Abgabe verschiedener therapeutischer Mittel 5 in gewünschter Dosis erreichen zu können.

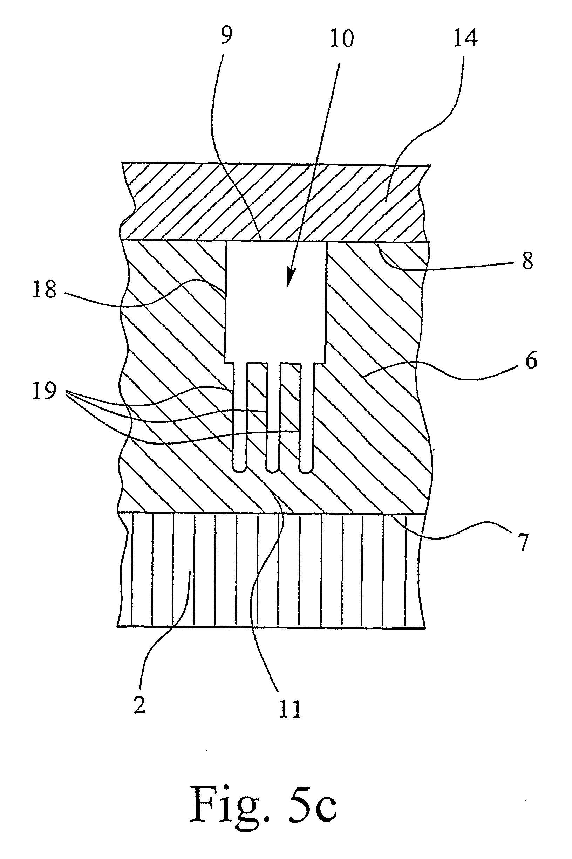

Fig. 5c zeigt in einer zu den Fig. 5a und 5b korrespondierenden Darstellung ein weiteres Ausführungsbeispiel des Implantats 1 mit nochmals modifizierten Hohlräumen 10 zur Erläuterung der unterschiedlichen Realisierungsmöglichkeiten. In diesem Fall weisen die Hohlräume 10 jeweils einen ersten, sich zur Oberfläche 8 der Deckschicht 6 hin öffnenden Abschnitt 18 und mehrere sich an den Abschnitt 18 an dem der Öffnung 9 entgegengesetzten Ende anschließende, in ihrem Durchmesser bzw. Querschnitt wesentlich gegenüber dem Abschnitt 18 reduzierte Abschnitte 19 auf. Die sich wurzel- bzw. fortsatzartig an die Abschnitte 18 der Hohlräume 10 anschließenden Abschnitte 19 bewirken aufgrund ihres verringerten Durchmessers bzw. Querschnitts eine beispielsweise gegenüber den Abschnitten 18 verlangsamte Abgabe bzw. Freisetzung eines aufgenommenen therapeutischen Mittels 5 im Vergleich zu der Abgabe bzw. Freisetzung von den Abschnitten 18. Gegebenenfalls können die Abschnitte 18 und die Abschnitte 19 der Hohlräume 10 auch mit unterschiedlichen therapeutischen Mitteln 5 versehen bzw. befüllt werden, wobei auch die Länge der Abschnitte 18 und 19, d. h. deren Erstreckung senkrecht zur Hauptebene bzw. Oberfläche 8 der Deckschicht 6, zueinander und insgesamt an ein gewünschtes Abgabeverhalten angepaßt werden können.

Um eine ausreichend hohe Dosis erreichen zu können, bedarf es einer bestimmten Menge des bzw. der therapeutischen Mittel(s) 5, das von den Hohlräumen 10 aufgenommen wird. Vorzugsweise sind etwa 10⁸ bis 10¹¹ Hohlräume pro cm² der Oberfläche 8 der Deckschicht 6 vorgesehen.

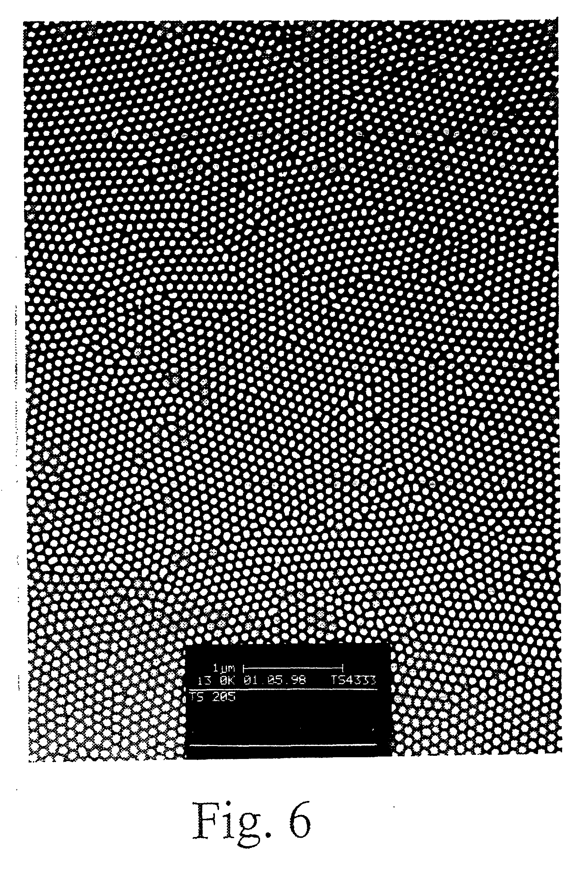

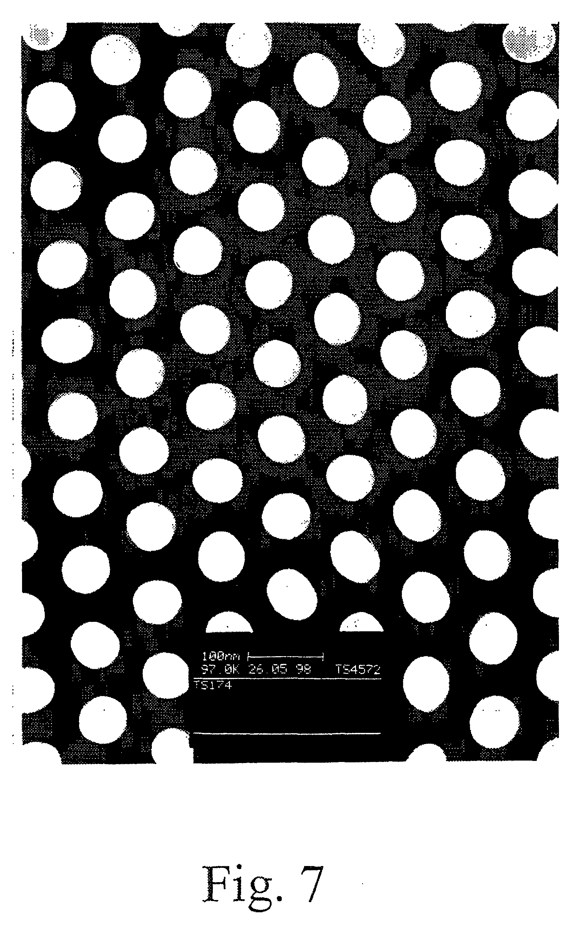

Fig. 6 und 7 stellen elektronenmikroskopische Aufnahmen einer Oberfläche einer Aluminiumoxidschicht bei unterschiedlicher Vergrößerung dar. Es ist deutlich erkennbar, wie gleichmäßig verteilt und ausgebildet die hell erscheinenden, rohrförmigen Hohlräume in der Aluminiumoxidschicht sind.

Gemäß einem besonders bevorzugten Ausführungsbeispiel ist radioaktives Material als therapeutisches Mittel 5 in den Hohlräumen 10 aufgenommen und insbesondere darin fixiert.

Die schematische, vergrößerte Schnittdarstellung eines Hohlraums 10 gemäß Fig. 5a veranschaulicht die Fixierung des radioaktiven Materials in den Hohlräumen 10 der Deckschicht 6. Die Wandung 12 des Hohlraums 10 ist mit Reaktionspartnern bzw. Komplexbildnern 13, vorzugsweise Thiolen oder Carboxylaten, die beispielsweise durch Silanisierung in den Hohlräumen 10 bzw. an deren Wandungen 12 gebunden sind, versehen, die beispielsweise über Mercaptogruppen das radioaktive Material in den Hohlräumen 10 binden bzw. fixieren.

Beispielsweise enthält das radioaktive Material radioaktives Technetium und/oder Rhenium, wobei insbesondere Technetium(V)- und/oder Rhenium(V) - Verbindungen mit schwefelhaltigen Liganden gebildet werden, die eine extrem hohe in vivo Stabilität aufweisen. Gemäß einem anderen Beispiel wird radioaktives Material in Form von ⁸⁶Y, ⁹⁰Y, ⁸⁹Sr, ¹⁵³Sm, ⁶⁴Cu, ⁶⁷Cu und/oder ¹⁰⁵Rh über (Poly-)Carboxylate in den Hohlräumen 10 fixiert, wobei die Carboxylate ihrerseits vorzugsweise durch Silanisierung in den Hohlräumen 10 gebunden sind.

Jedoch können auch andere Radionuklide, beispielsweise auch Anionen, wie Iod, als radioaktives Material in den Hohlräumen 10 fixiert und insbesondere mittels geeigneter Reaktionspartner, beispielsweise Edelmetallen, insbesondere Silber, chemisch gebunden werden. Beispielhaft ist hier weiter die Bindung von insbesondere flüssig eingebrachtem, radioaktivem Material 5 in Form von ¹²⁰I, ¹²³I, ¹²⁴I, ¹²⁵I, ¹³¹I und/oder ²¹¹At und dessen Bindung über Silber in den Hohlräumen 10 zu nennen, wobei das Silber seinerseits beispielsweise durch (Poly-)Carboxylate gebunden ist, die ihrerseits vorzugsweise durch Silanisierung in den Hohlräumen 10 gebunden sind.

Vorzugsweise enthält das radioaktive Material verschiedene Radionuklide in einem gewünschten Verhältnis, so daß eine in räumlicher und/oder zeitlicher Hinsicht optimale Dosis aufgrund der unterschiedlichen Eigenschaften der verschiedenen Radionuklide erreicht wird. Dies ist bei der vorschlagsgemäßen Einbringung des radioaktiven Materials in die Hohlräume 10 verhältnismäßig einfach möglich, da beispielsweise verschiedene Radioisotope und/oder verschiedene Radionuklide mit unterschiedlichem Halbwertszeiten, Energien und/oder Strahlungsarten (α, β+, β-, γ) beispielsweise miteinander vermischt oder vermengt in die Hohlräume 10 eingebracht und dort beispielsweise über entsprechend ausgewählte Komplexbildner 13 fixiert werden können.

Alternativ können verschiedene Radionuklide auch nacheinander, also beispielsweise schichtweise, in die Hohlräume 10 eingebracht und mittels entsprechender oder beispielsweise selektiver Komplexbildner 13 fixiert werden.

Alternativ oder zusätzlich ist es möglich, die Hohlräume 10 nicht vollständig mit radioaktivem Material zu füllen, sondern beispielsweise zusätzliches Füllmaterial zur Stabilisierung und/oder einem Verschließen der Öffnungen 9 bei nur teilweiser Befüllung mit radioaktivem Material 5 zuzugeben.

Die außerdem mögliche unterschiedliche Füllung der Hohlräume 10 mit radioaktivem Material zur Modifizierung der Dosisverteilung wurde bereits erwähnt.

Insbesondere ist der Durchmesser der Hohlräume 10 und/oder der Öffnungen 9 so gewählt, daß die Blutbestandteile bzw. die üblicherweise im Blut 4 vorhandenen Moleküle, die verhältnismäßig groß sind, aufgrund ihrer Größe nicht durch die Öffnungen 9 in die Hohlräume 10 eindringen können. Dies kann durch einen Durchmesser der Öffnungen 9 von etwa 5 bis max. 100 nm sichergestellt werden.

Um eine ausreichend hohe Dosis erreichen zu können, bedarf es einer bestimmten Menge an radioaktivem Material, das von den Hohlräumen 10 aufgenommen wird. Vorzugsweise sind etwa 10⁸ bis 10¹¹ Hohlräume 10 pro cm² der Oberfläche 8 der Deckschicht 6 vorgesehen.

Schließlich ist darauf hinzuweisen, daß die vorschlagsgemäße Anordnung des radioaktiven Materials in Hohlräumen 10 einer vorschlagsgemäßen Deckschicht 6 nicht auf Implantate beschränkt, sondern auch bei sonstigen Bauelementen bzw. radioaktiven Strahlern mit gewünschten radioaktiven Eigenschaften einsetzbar ist.

## Patentansprüche

1. Implantat (1) mit einem Träger (2), insbesondere aus Metall, und mit mindestens einem therapeutischen Mittel (5), wobei der Träger (2) eine insbesondere in mit Körpergewebe und/oder -flüssigkeiten in Kontakt tretenden Bereichen angeordnete, zumindest abschnittsweise ausgebildete Deckschicht (6) mit einer Vielzahl von durch elektrolytische Oxidierung erzeugten Hohlräumen (10) mit separaten Öffnungen (9) zur Oberfläche (8) der Deckschicht (6) zur Aufnahme des mindestens einen therapeutischen Mittels (5) aufweist,
**dadurch gekennzeichnet,**
**daß** die Deckschicht (6) zumindest im wesentlichen aus elektrolytisch oxidiertem Aluminium-, Magnesium-, Tantal-, Eisen- und/oder Wolframoxid besteht,
**daß** die Öffnungen (9) und/oder die Hohlräume (10) zumindest im wesentlichen gleichförmig ausgebildet sind und
**daß** die Hohlräume (10) und/oder deren Öffnungen (9) einen Querschnitt bzw. eine Öffnungsfläche mit einem größten oder mittleren Durchmesser von im Mittel maximal 100 nm aufweisen.

2. Implantat nach Anspruch 1, **dadurch gekennzeichnet, daß** der Träger (2) mit einer elektrolytisch aufgebrachten bzw. oxidierten Deckschicht (6) und/oder mit einer im Plasmabeschichtungsverfahren, insbesondere aus der Dampfphase, aufgebrachten Deckschicht (6) versehen ist und/oder daß die Deckschicht (6) durch eine insbesondere oxidierte Oberflächenschicht des Trägers (2) gebildet ist.

3. Implantat nach einem der voranstehenden Ansprüche, **dadurch gekennzeichnet,**
**daß** die Deckschicht (6) nur auf außenseitigen oder sowohl auf außenseitigen als auch auf innenseitigen Oberflächenabschnitten des Trägers (2) ausgebildet ist,
**daß** die Deckschicht (6) im wesentlichen gleichmäßig dick ausgebildet ist,
**daß** die Dicke der Deckschicht (6) zumindest im wesentlichen höchstens 1,5 µm, vorzugsweise maximal 200 nm und insbesondere etwa 30 bis 150 nm, beträgt, und/oder
**daß** die Deckschicht (6) eine insbesondere für Körperflüssigkeiten undurchlässige, vorzugsweise die gesamte Oberfläche (7) des Trägers (2) bedeckende Sperrschicht (11) bildet.

4. Implantat nach einem der voranstehenden Ansprüche, **dadurch gekennzeichnet,**
**daß** die Hohlräume (10) zumindest im wesentlichen gleichmäßig ausgebildet sind und/oder daß die Hohlräume (10) zumindest im wesentlichen länglich, insbesondere röhrenartig, ausgebildet und/oder jeweils zumindest einseitig verschlossen sind,
**daß** die Hohlräume (10) voneinander beabstandet bzw. getrennt ausgebildet sind und/oder daß die Hohlräume (10) zumindest im wesentlichen parallel zueinander und/oder zumindest im wesentlichen senkrecht zur Oberfläche (7, 8) der Deckschicht (6) und/oder des Trägers (2) verlaufen,
**daß** die Deckschicht (6) Hohlräume (10) mit unterschiedlichen Querschnitten und/oder Volumina und/oder Öffnungsflächen zur Oberfläche (8) der Deckschicht (6) aufweist und/oder daß die Hohlräume (10) jeweils Abschnitte mit unterschiedlichen bzw. zu- und/oder abnehmenden Querschnitten aufweisen und/oder verzweigt sind, und/oder
**daß** die Hohlräume (10) ausschließlich in der Deckschicht (6) ausgebildet sind.

5. Implantat nach einem der voranstehenden Ansprüche, **dadurch gekennzeichnet,**
**daß** die Hohlräume (10) und/oder deren Öffnungen (9) über zumindest einen Teil der Oberfläche (8) der Deckschicht (6) verteilt angeordnet sind und/oder daß die Hohlräume (10) bzw. deren Öffnungen (9) mit einer Flächendichte von 10⁸ bis 10¹¹/cm² über die Oberfläche (8) der Deckschicht (6) verteilt sind,
**daß** die Summe der Querschnittsflächen der Fläche der Öffnungen (9) höchstens 50% der Oberfläche (8) der Deckschicht (6) beträgt und/oder
**daß** die Öffnungen (9) zumindest im wesentlichen die gleiche Querschnittsfläche und/oder zumindest im wesentlichen den gleichen minimalen, mittleren und/oder maximalen Durchmesser aufweisen, und/oder
**daß** die Hohlräume (10) und/oder deren Öffnungen (9) einen Querschnitt bzw. eine Öffnungsfläche mit einem größten oder mittleren Durchmesser von im Mittel maximal 50 nm, insbesondere etwa 25 nm oder weniger, aufweisen.

6. Implantat nach einem der voranstehenden Ansprüche, **dadurch gekennzeichnet, daß** das Implantat (1) als Stent ausgebildet ist.

7. Implantat nach einem der voranstehenden Ansprüche, **dadurch gekennzeichnet,**
**daß** das therapeutische Mittel (5) zumindest teilweise an Wandungen (12) der Hohlräume (10), insbesondere über Komplexbildner (13), chemisch gebunden ist,
**daß** das therapeutische Mittel (5) derart in den Hohlräumen (10) gebunden bzw. fixiert ist, daß dieses bei Überschreiten einer vorbestimmten Temperatur und/oder durch körperspezifische Stoffe, Körperflüssigkeiten, Enzyme oder Aktivierungsstoffe oder durch Einwirkung einer sonstigen Aktivierung, insbesondere Laser oder Ultraschall, vom Implantat (1) im implantierten Zustand abgeben wird, und/oder
**daß** das Implantat (1) mindestens zwei in den Hohlräumen (10) derart aufgenommene therapeutische Mittel (5) aufweist, daß die Mittel (5) nacheinander und/oder mit unterschiedlicher Geschwindigkeit und/oder in unterschiedlicher Menge im implantierten Zustand abgebbar sind.

8. Implantat nach einem der voranstehenden Ansprüche, **dadurch gekennzeichnet, daß** das Implantat (1) als therapeutisches Mittel (5) radioaktives Material umfaßt, das eine insbesondere vorbestimmte Menge mindestens eines Radionukleids enthält und das in den Hohlräumen (10) aufgenommen und insbesondere darin fixiert ist.

9. Implantat nach Anspruch 8, **dadurch gekennzeichnet,**
**daß** das radioaktive Material an Wandungen (12) der Hohlräume (10), insbesondere über Komplexbildner (13), wie Thiole, chemisch gebunden ist,
**daß** das radioaktive Material radioaktives Rhenium und/oder Technetium enthält, das insbesondere über Schwefelgruppen in den Hohlräumen (10) fixiert ist, und/oder
**daß** das radioaktive Material verschiedene Radionuklide, insbesondere mit unterschiedlichen Halbwertszeiten, Strahlungsarten und/oder Energien, in vorbestimmten Mengen und Verhältnissen umfaßt.

10. Implantat nach einem der voranstehenden Ansprüche, **dadurch gekennzeichnet, daß** die Deckschicht (6) bzw. die Öffnungen (9) der Hohlräume (10) durch eine Abdeckschicht (14), vorzugsweise aus Gold, zumindest temporär überdeckt bzw. verschlossen sind.

11. Verfahren zur Herstellung eines nach einem der Ansprüche 1 bis 10 ausgebildeten Implantats (1),
**dadurch gekennzeichnet,**
**daß** das Implantat (1) zur Evakuierung der Hohlräume (10) unter Unterdruck gesetzt und anschließend das therapeutische Mittel (5) und/oder ein das therapeutische Mittel (5) in den Hohlräumen (10) bindendes Bindungsmittel (13) den Hohlräumen (10), insbesondere durch Eintauchen des Implantats (1) in das therapeutische Mittel (5) bzw. das Bindungsmittel (13), zugeführt und der Druck wieder normalisiert wird und/oder
**daß** das therapeutische Mittel (5) und/oder ein das therapeutische Mittel (5) in den Hohlräumen (10) bindendes Bindungsmittel (13) mittels Ultraschall, insbesondere bei in das therapeutische Mittel (5) bzw. in das Bindungsmittel (13) eingetauchtem Implantat (1), in die Hohlräume (10) eingebracht wird.

12. Verfahren zur Herstellung eines nach einem der Ansprüche 1 bis 10 ausgebildeten Implantats (1),
**dadurch gekennzeichnet,**
**daß** die Hohlräume (10) bzw. deren Wandungen (12) mit Komplexbildnern (13) bzw, das therapeutische Mittel (5) chemisch bindenden Reaktionspartnern beschichtet bzw. versehen werden und anschließend das therapeutische Mittel (5) in die Hohlräume (10) eingebracht wird.

13. Verfahren nach Anspruch 12, **dadurch gekennzeichnet, daß** das therapeutische Mittel (5) in die Hohlräume (10) mittels Unterdruck eingebracht wird, daß radioaktives Material als therapeutisches Mittel (5) in den Hohlräumen (10) durch Komplexbildung und/oder durch chemische Bindung fixiert wird, und/oder daß die Hohlräume (10) bzw. die Deckschicht (6) mit einer Abdeckschicht (14), beispielsweise aus Gold, überdeckt werden bzw. wird.

14. Verwendung eines Implantats (1) mit einem Träger (2), insbesondere aus Metall, der eine insbesondere in mit Körpergewebe und/oder -flüssigkeiten in Kontakt tretenden Bereichen angeordnete, zumindest abschnittsweise ausgebildete Deckschicht (6) aufweist, die eine Vielzahl von durch elektrolytische Oxidierung erzeugten Hohlräumen (10) mit separaten Öffnungen (9) zur Oberfläche (8) der Deckschicht (6) aufweist und die zumindest im wesentlichen aus Aluminium-, Magnesium-, Tantal-, Eisen- und/oder Wolframoxid besteht, wobei die Öffnungen (9) und/oder die Hohlräume (10) zumindest im wesentlichen gleichförmig ausgebildet sind, zur Bereitstellung eines therapeutischen Mittels (5) im menschlichen oder tierischen Körper,
**dadurch gekennzeichnet,**
**daß** die Hohlräume (10) und/oder deren Öffnungen (9) einen Querschnitt bzw. eine Öffnungsfläche mit einem größten oder mittleren Durchmesser von im Mittel maximal 100 nm aufweisen und
**daß** das mindestens eine therapeutische Mittel (5) vor dem Implantieren des Implantats (1) in die Hohlräume (10) eingebracht wird.

## Claims

1. Implant (1) with a support (2), in particular in metal, and with at least one therapeutic agent (5), wherein the support (2) comprises an at least partially formed covering layer (6) preferably arranged in areas coming into contact with body tissue and/or body liquids having a plurality of cavities (10) formed by electrolytic oxidization with separate openings (9) open to the surface (8) of the covering layer (6) for absorbing the at least one therapeutic agent (5),
**characterized in**
**that** the covering layer (6) at least essentially consists of electrolytically oxidized aluminum oxide, magnesium oxide, tantalum oxide, iron oxide and/or tungsten oxide,
**that** the openings (9) and/or the cavities (10) are formed at least essentially congruously, and
**that** the cavities (10) and/or their openings (9) comprise a cross-section or an opening area with a largest or average diameter of a maximum of 100 nm on average.

2. Implant according to claim 1, **characterized in that** the support (2) is provided with an electrolytically applied or oxidized covering layer (6) and/or a covering layer (6) applied in the plasma deposition method, in particular from the vapor phase, and/or that the covering layer (6) is formed by a preferably oxidized surface layer of the support (2).

3. Implant according any one of the preceding claims, **characterized in**
**that** the covering layer (6) is only formed on outside surface portions or both outside and inside surface portions of the support (2),
**that** the covering layer (6) is essentially formed with uniform thickness,
**that** the thickness of the covering layer (6) is at least essentially at most 1.5 µm, preferably a maximum of 200 nm and in particular about 30 to 150 nm, and/or
**that** the covering layer (6) forms a barrier layer (11) in particular impermeable for body fluids preferably covering the whole surface (7) of the support (2).

4. Implant according to any one of the preceding claims, **characterized in**
**that** the cavities (10) are at least essentially homogeneously formed and/or that the cavities (10) are formed at least essentially longitudinally, in particular tube-like, and/or are each closed at least on one side,
**that** the cavities (10) are formed spaced or separated from each other and/or that the cavities (10) extend at least essentially parallel to each other and/or at least essentially perpendicularly to the surface (7, 8) of the covering layer (6) and/or the support (2),
**that** the covering layer (6) comprises cavities (10) with different cross-sections and/or volumes and/or opening areas to the surface (8) of the covering layer (6) and/or that the cavities (10) each comprise sections with different or increasing and/or decreasing cross-sections and/or are branched, and/or
**that** the cavities (10) are formed in the covering layer (6) exclusively.

5. Implant according to any one of the preceding claims, **characterized in**
**that** the cavities (10) and/or their openings (9) are distributedly arranged over at least a portion of the surface (8) of the covering layer (6) and/or that the cavities (10) or their openings (9) are distributed with a surface density of 10⁸ to 10¹¹/cm² over the surface (8) of the covering layer (6),
**that** the sum of the cross-sectional opening areas of the area of the openings (9) is at most 50 % of the surface (8) of the covering layer (6), and/or
**that** the openings (9) comprise at least essentially the same cross-sectional opening area and/or at least essentially the same minimum, average and/or maximum diameter, and/or
the cavities (10) and/or their openings (9) comprise a cross-section or an opening area with a largest or average diameter of maximum 50 nm on average, in particular about 25 nm or less.

6. Implant according to any of one the preceding claims, **characterized in that** the implant (1) is formed as a stent.

7. Implant according to any one of the preceding claims, **characterized in**
**that** the therapeutic agent (5) is fixed or bound chemically, at least partly to the walls (12) or the cavities (10), in particular via complexing agents (13),
**that** the therapeutic agent (5) is fixed or bound in the cavities (10) so that it is released from the implant (1) in the implanted condition when a predetermined temperature is exceeded and/or by body-specific substances, body fluids, enzymes or activation substances or due to some other activation, in particular by laser or ultrasound, and/or
**that** the implant (1) comprises at least two therapeutic agents (5) absorbed in the cavities (10) such that the agents (5) are releasable in succession and/or with a different rate and/or in a different amount in the implanted condition.

8. Implant according to any one of the preceding claims, **characterized in that** the implant (1) includes radioactive material as therapeutic agent (5) which contains a preferably predetermined amount of at least one radionuclide and is absorbed in the cavities (10) and in particular fixed therein.

9. Implant according to claim 8, **characterized in**
**that** the radioactive material is chemically bound or fixed to walls (12) of the cavities (10), in particular via complexing agents (13), like thiols,
**that** the radioactive material contains radioactive rhenium and/or technetium, that is fixed in the cavities (10) in particular via sulphur groups, and/or
**that** the radioactive material includes various radionuclides in predetermined amounts and ratios, in particular with different half-life values, types of radiation and/or energies.

10. Implant according to any one of the preceding claims, **characterized in that** the covering layer (6) or the openings (9) of the cavities (10) are at least temporarily closed or covered by a cover layer (14), preferably in gold.

11. Method for producing an implant (1) formed according to any one of claims 1 to 10,
**characterized in**
**that** the implant (1) is put under low pressure in order to evacuate the cavities (10) and then the therapeutic agent (5) and/or a binder (13) binding the therapeutic agent (5) in the cavities (10) is introduced into the cavities (10), in particular by dipping the implant (1) into the therapeutic agent (5) or the binder (13), and the pressure is returned to normalcy, and/or
**that** the therapeutic agent (5) and/or a binder (13) binding the therapeutic agent (5) in the cavities (10) is incorporated in the cavities (10) by means of ultrasound, in particular when the implant (1) is dipped into the therapeutic agent (5) or in the binder (13).

12. Method for producing an implant (1) formed according to any one of claims 1 to 10,
**characterized in**
**that** the cavities (10) or their walls (12) are provided or coated with complexing agents (13) or reaction partners chemically binding the therapeutic agent (5) and that the therapeutic agent (5) is then incorporated in the cavities (10).

13. Method according to claim 12, **characterized in that** the therapeutic agent (5) is incorporated in the cavities (10) by means of low pressure, that radioactive material as a therapeutic agent (5) is fixed in the cavities (10) via formation of complexes and/or via chemical binding, and/or that the cavities (10) or the covering layer (6) are respectively covered with a cover layer (14), for example of gold.

14. Use of an implant (1) with a support (2), in particular of metal, which comprises an at least partially formed covering layer (6), preferably arranged in areas coming into contact with body tissue and/or body fluids, which comprises a plurality of cavities (10) formed by electrolytic oxidization with separate openings (9) to the surface (8) of the covering layer (6) and which at least essentially consists of aluminum oxide, magnesium oxide, tantalum oxide, iron oxide and/or tungsten oxide, wherein the openings (9) and/or the cavities (10) comprise at least essentially the same shape, for providing a therapeutic agent (5) in the human or animal body,
**characterized in**
**that** the cavities (10) and/or their openings (9) comprise a cross-section or an opening area with a largest or average diameter of maximum 100 nm on average, and
**that** the at least one therapeutic agent (5) is incorporated in the cavities (10) before implanting the implant (1).

## Revendications

1. Implant (1) comportant un support (2), en particulier en métal, et au moins un agent thérapeutique (5), dans lequel le support (2) présente une couche de protection (6) réalisée au moins sous la forme de morceaux détachés, disposée en particulier dans des zones entrant en contact avec un tissu corporel et/ou des fluides corporels, ladite couche comprenant une multitude d'espaces creux (10) réalisés par oxydation électrolytique, lesdits espaces comprenant des ouvertures séparées (9) donnant sur la surface (8) de la couche de protection (6) pour la réception dudit ou desdits agents thérapeutiques (5),
**caractérisé**
**en ce que** la couche de protection (6) est constituée au moins de manière essentielle d'oxyde d'aluminium, de magnésium, de tantale, de fer et/ou de tungstène, l'oxydation étant réalisée par voie électrolytique,
**en ce que** les ouvertures (9) et/ou les espaces creux (10) sont réalisés au moins de manière essentielle pour être uniformes,
**en ce que** les espaces creux (10) et/ou leurs ouvertures (9) présentent une section transversale, respectivement une surface d'ouverture comprenant un diamètre maximal ou moyen qui s'élève en moyenne au maximum à 100 nm.

2. Implant selon la revendication 1, **caractérisé en ce que** le support (2) est muni d'une couche de protection (6) appliquée, respectivement oxydée par voie électrolytique et/ou d'une couche de protection (6) appliquée dans le procédé d'enduction au plasma, en particulier à partir de la phase vapeur et/ou **en ce que** la couche de protection (6) est formée par une couche superficielle du support (2) en particulier oxydée.

3. Implant selon l'une quelconque des revendications précédentes, **caractérisé**
**en ce que** la couche de protection (6) est réalisée uniquement sur des sections superficielles externes ou aussi bien sur des sections superficielles externes que sur des sections superficielles internes du support (2),
**en ce que** la couche de protection (6) est réalisée de telle sorte qu'elle possède une épaisseur essentiellement uniforme,
**en ce que** l'épaisseur de la couche de protection (6) s'élève au moins de manière essentielle au maximum à 1,5 µm, de préférence au maximum à 200 nm et en particulier d'environ 30 à 150 nm, et/ou
**en ce que** la couche de protection (6) forme une couche d'arrêt (11), en particulier imperméable pour les fluides corporels, et recouvrant de préférence la surface totale (7) du support (2).

4. Implant selon l'une quelconque des revendications précédentes, **caractérisé**
**en ce que** les espaces creux (10) sont réalisés au moins de manière essentielle pour être uniformes et/ou en ce que les espaces creux (10) sont réalisés au moins de manière essentielle sous forme oblongue, en particulier sous forme tubulaire et/ou sont respectivement obturés, au moins d'un côté,
**en ce que** les espaces creux (10) sont réalisés pour être espacés, respectivement séparés les uns des autres et/ou en ce que les espaces creux (10) s'étendent au moins de manière essentielle en position réciproquement parallèle et/ou s'étendent au moins de manière essentielle perpendiculairement à la surface (7, 8) de la couche de protection (6) et/ou du support (2),
**en ce que** la couche de protection (6) présente des espaces creux (10) comprenant des sections transversales différentes et/ou des volumes différents et/ou des surfaces d'ouvertures différentes en direction de la surface (8) de la couche de protection (6) et/ou en ce que les espaces creux (10) présentent respectivement des morceaux détachés comprenant des sections transversales différentes, respectivement qui augmentent et/ou qui diminuent et/ou sont ramifiés, et/ou
**en ce que** les espaces creux (10) sont réalisés exclusivement dans la couche de protection (6).

5. Implant selon l'une quelconque des revendications précédentes, **caractérisé**
**en ce que** les espaces creux (10) et/ou leurs ouvertures (9) sont disposés en étant répartis sur au moins une partie de la surface (8) de la couche de protection (6) et/ou en ce que les espaces creux (10), respectivement leurs ouvertures (9), sont répartis avec une densité superficielle de 10⁸ à 10¹¹/cm² sur la surface (8) de la couche de protection (6),
**en ce que** la somme des superficies des sections des ouvertures (9) représente au maximum 50 % de la surface (8) de la couche de protection (6) et/ou
**en ce que** les ouvertures 9 présentent au moins de manière essentielle la même superficie de section et/ou au moins de manière essentielle le même diamètre minimal, moyen et/ou maximal, et/ou
**en ce que** les espaces creux (10) et/ou leurs ouvertures (9) présentent une section transversale, respectivement une surface d'ouverture comprenant un diamètre maximal ou moyen qui s'élève en moyenne au maximum à 50 nm, en particulier à environ 25 nm ou moins.

6. Implant selon l'une quelconque des revendications précédentes, **caractérisé en ce que** l'implant (1) est réalisé sous la forme d'un extenseur pour la désobstruction des artères.

7. Implant selon l'une quelconque des revendications précédentes, **caractérisé**
**en ce que** l'agent thérapeutique (5) est lié par voie chimique, en particulier via des formateurs de complexes (13), au moins en partie aux parois (12) des espaces creux (10),
**en ce que** l'agent thérapeutique (5) est lié, respectivement fixé dans les espaces creux (10) de telle sorte que, lorsqu'une température prédéterminée est dépassée et/ou sous l'action de substances spécifiques au corps, de fluides corporels, d'enzymes ou de substances d'activation ou encore sous l'influence d'une autre activation, en particulier par laser ou par ultrasons, il est libéré de l'implant (1) à l'état implanté, et/ou
**en ce que** l'implant (1) présente au moins deux agents thérapeutiques (5) logés dans les espaces creux (10) de telle sorte que les agents (5) puissent être libérés l'un après l'autre et/ou avec une vitesse différente et/ou en une quantité différente à l'état implanté.

8. Implant selon l'une quelconque des revendications précédentes, **caractérisé en ce que** l'implant (1) comprend, à titre d'agent thérapeutique (5) une matière radioactive qui contient une quantité en particulier prédéterminée au moins d'un radionucléide et qui est logée et en particulier fixée dans les espaces creux (10).

9. Implant selon la revendication 8, **caractérisé**
**en ce que** la matière radioactive est liée par voie chimique aux parois (12) des espaces creux 10, en particulier via des formateurs de complexes (13) tels que des thiols,
**en ce que** la matière radioactive contient du rhénium et/ou du technétium radioactif qui est fixé en particulier via des groupes de soufre dans les espaces creux (10), et/ou
**en ce que** la matière radioactive comprend différents radionucléides, en particulier avec des temps de demi-vie différents, avec des types de rayonnements différents et/ou avec des énergies différentes, dans des quantités et dans des rapports prédéterminés.

10. Implant selon l'une quelconque des revendications précédentes, **caractérisé en ce que** la couche de protection (6), respectivement les ouvertures (9) des espaces creux (10) sont recouvertes, respectivement obturées, au moins de manière temporaire à l'aide d'une couche de recouvrement (14), de préférence constituée d'or.

11. Procédé pour la fabrication d'un implant (1) réalisé conformément à l'une quelconque des revendications 1 à 10, **caractérisé**
**en ce qu'**on soumet l'implant (1) à une pression négative pour faire le vide dans les espaces creux (10) et on achemine ensuite l'agent thérapeutique (5) et/ou un liant (13) qui lie l'agent thérapeutique (5) dans les espaces creux (10), en particulier par imprégnation de l'implant (1) dans l'agent thérapeutique (5), respectivement dans le liant (13) et on rétablit ensuite la pression normale, et/ou
**en ce qu'**on introduit l'agent thérapeutique (5) et/ou un liant (13) qui lie l'agent thérapeutique (5) dans les espaces creux (10), à l'aide d'ultrasons, en particulier en introduisant, dans les espaces creux (10), l'implant (1) imprégné dans l'agent thérapeutique (5), respectivement dans le liant (13).

12. Procédé pour la fabrication d'un implant (1) réalisé conformément à l'une quelconque des revendications 1 à 10, **caractérisé**
**en ce qu'**on enduit, respectivement on munit les espaces creux (10), respectivement leurs parois (12) avec des formateurs de complexes (13), respectivement avec des partenaires réactionnels qui lient l'agent thérapeutique (5) par voie chimique et on introduit ensuite l'agent thérapeutique (5) dans les espaces creux (10).

13. Procédé selon la revendication 12, **caractérisé en ce qu'**on introduit l'agent thérapeutique (5) dans les espaces creux (10) en utilisant une pression négative, **en ce qu'**on fixe la matière radioactive à titre d'agent thérapeutique (5) dans les espaces creux (10) par formation de complexes et/ou par liaison chimique et/ou **en ce qu'**on recouvre les espaces creux (10), respectivement la couche de protection (6) avec une couche de recouvrement (14), par exemple constituée d'or.

14. Utilisation d'un implant (1) comprenant un support (2), en particulier en métal, qui présente une couche de protection (6) réalisée au moins sous la forme de morceaux détachés, disposée en particulier dans des zones entrant en contact avec un tissu corporel et/ou des fluides corporels, ladite couche comprenant une multitude d'espaces creux (10) réalisés par oxydation électrolytique, lesdits espaces comprenant des ouvertures séparées (9) donnant sur la surface (8) de la couche de protection (6), et étant constituée au moins de manière essentielle d'oxyde d'aluminium, de magnésium, de tantale, de fer et/ou de tungstène, les ouvertures (9) et/ou les espaces creux (10) étant réalisés au moins de manière essentielle pour être uniformes, pour procurer un agent thérapeutique 5 dans le corps humain ou animal,
**caractérisée**
**en ce que** les espaces creux (10) et/ou leurs ouvertures (9) présentent une section transversale, respectivement une surface d'ouverture comprenant un diamètre maximal ou moyen qui s'élève en moyenne au maximum à 100 nm et
**en ce qu'**on introduit au moins un agent thérapeutique (5), avant l'implantation de l'implant (1), dans les espaces creux (10).
